# EUROPEAN PATENT SPECIFICATION

(11) **EP 4 366 797 B1**
(45) Date of publication and mention of the grant of the patent: **19.08.2026**
(21) Application number: 22740529.7
(22) Date of filing: 07.07.2022
(51) Int. Cl.: A61M 5/142, A61M 5/172

(54) **A SYSTEM FOR THE CONTROLLED ADMINISTRATION OF A SUBSTANCE WITH AN IMPLANTABLE INFUSION DEVICE PROVIDED WITH AN IMPROVED DOCKING GROUP FOR RELIABLY DOCKING AN INGESTIBLE SUBSTANCE CARRIER**
SYSTEM ZUR KONTROLLIERTEN VERABREICHUNG EINER SUBSTANZ MIT EINER IMPLANTIERBAREN INFUSIONSVORRICHTUNG MIT VERBESSERTER ANDOCKGRUPPE ZUM ZUVERLÄSSIGEN ANDOCKEN EINES EINNEHMBAREN SUBSTANZTRÄGERS
SYSTÈME POUR L'ADMINISTRATION RÉGULÉE D'UNE SUBSTANCE AVEC UN DISPOSITIF DE PERFUSION IMPLANTABLE POURVU D'UN GROUPE D'ACCUEIL AMÉLIORÉ POUR ARRIMER DE MANIÈRE FIABLE UN SUPPORT DE SUBSTANCE INGÉRABLE

(30) Priority: 08.07.2021 IT 202100017999
(43) Date of publication of application: 15.05.2024
(73) Proprietor: Scuola Superiore di Studi Universitari e di Perfezionamento Sant'Anna, 56127 Pisa (IT); Lifecare Laboratory GmbH, 55128 Mainz (DE)
(72) Inventor: AL-HADDAD, Hind, 56127 Pisa (IT); TAMADON, Izadyar, 56127 Pisa (IT); GUARNERA, Daniele, 56127 Pisa (IT); MENCIASSI, Arianna, 56025 Pontedera (Pisa) (IT); DARIO, Paolo, 56043 Lorenzana (Pisa) (IT); PFÜTZNER, Andreas, 55128 Mainz (DE); VISTOLI, Fabio, 56127 Pisa (IT); IACOVACCI, Veronica, 04010 Sezze (Latina) (IT); RICOTTI, Leonardo, 56048 Volterra (Pisa) (IT)
(74) Representative: Soldatini, Andrea
(86) International application number: PCT/IB2022/056271
(87) International publication number: WO 2023/281428

(56) References cited:
- WO-A1-2012/011132
- WO-A1-2012/087668

## Description

### Field of the Invention

The present invention generally regards the controlled administration of substances through infusion devices implanted in the human body and more particularly has as its object an improved system for the controlled administration of a substance such as a drug, a hormone or a hormone complex and the like for which other administration modes result unsatisfactory or ineffective. More specifically, the invention is directed to such a system comprising an implanted infusion device provided with an improved docking group for reliably docking an ingestible substance carrier.

### Background of the Invention

A known system for the controlled administration of a substance from a human-body-implanted infusion device is disclosed in patent publication n. WO2012011132. This document discloses a system that comprises:
- an implantable monitoring unit for the monitoring of the target substance;
- an infusion device of the substance implantable in the peritoneal cavity comprising a communication and control unit to manage the data arriving from the monitoring unit for the substance release;
- a carrier of the target substance, to be ingested in order to reach passively the intestinal lumen, made of perforable material, resistant to the gastric acids and with metallic inserts;
- a refilling station to refill the above-mentioned infusion device and associated to the latter, the refilling station comprising a docking group for the magnetic docking of the carrier and a punching unit for drawing the substance from the carrier.

The refilling of the drug is devised to take place periodically through the ingestion of a carrier in the form of a capsule containing the desired substance. The disclosure is particularly suitable for insulin delivery, but it can be extended to other drugs as well. Once ingested by the patient, the carrier passively travels along the digestive system up to a certain intestinal loop, where the infusion group is implanted and where the carrier is magnetically docked by the docking group of the refill device.

The docking group presents two ferromagnetic elements enclosing a diametrically magnetized permanent magnet, separated by two non-ferromagnetic inserts. The magnet can rotate around its axis of 90° (thanks to a motor and a gear transmission) to switch from a deactivated condition to an activated one. The permanent magnet is oriented with respect to the two non-ferromagnetic elements so that, when the poles of the magnet are turned towards them, the field lines are shut from the north to the south poles on a short path through the close ferromagnetic material, with no magnetic field outside. By rotating the magnet by 90°, its poles are separated by the two non-ferromagnetic elements through which the field lines cannot pass. The preferential path then becomes the longer path through the ferromagnetic elements. This results in a corresponding polarization of the ends facing in the duodenal lumen.

The capsule consists of a cylindrical body that delimits an internal chamber for the substance storage. The capsule is made of a polymeric material, resistant to gastric acids, and is punchable by a needle. A metallic magnetizable structure, possibly covered with a polymeric coating avoiding the direct contact of the metal with the anatomical environment, is included in the carrier body, at a central portion thereof.

When the capsule reaches the duodenum and is close to the implanted device, it transmits a position signal to the control unit which activates the motor for the 90° rotation of the magnet. In this way, a magnetic field insists between the two ends of the pole to attract the arriving carrier, due to the metallic structure embedded on it. Once the capsule is perfectly docked, the control unit activates a linear motor able to eject a needle out to draw the substance from the carrier, with the help of a vacuum pump. The anchoring of the carrier to the device is allowed by the attraction force exerted by the docking circuit.

### Summary of the Invention

The present invention has its context in the observation of the above-mentioned system and the recognition of certain critical problems affecting its performance; this brought to the system for the controlled administration of a substance with an implanted infusion device and an improved docking of an ingestible substance carrier according to the invention, having the essential features of attached claim 1. A further aspect of the invention is in connection with a special configuration of the substance carrier, as per the contents of the relevant attached claims. Advantageous embodiments of the invention are defined by the other claims.

A first problem that was devised is in connection with the effectiveness and reliability of the needle punching action once the substance carrier is docked. The support at the central region of the carrier, and the needle punching occurring at a point displaced towards one end creates a torque opposing docking that may cause the rotation of the capsule with the risk of detachment. This problem is made more serious considering that the intensity of the magnetic attraction force is drastically reduced by the presence of tissue layers between the docking circuit and the substance carrier, thus increasing the risk of undesired detachment due to needle-induced rotation.

The docking at the central region can also result in a defective precision of the docking position, with consequent position uncertainty and thus possible unpredictable variations in the docking (and punching) behavior. Even when a docking system is used based on two magnetic units, which can capture two rings of the carrier once activated at the same time by the single permanent magnet, the attraction of the capsule may vary, depending on which ferromagnetic ring is attracted first. Possible scenarios include the docking of only a single unit of the magnetic cage with either of the rings, and a consequent seriously off-centered docking that may even prevent the punching itself or increase the above-mentioned detaching torque. The only way to prevent such problem could be to activate the docking system exactly at the moment in which the capsule is centered with respect to the magnetic docking cage. However, given the variability of the anatomic site and the peristaltic movements that drive the capsule advancement, this strategy is very fragile and poorly reliable.

The above described docking aspects have clearly a counterpart in the structure of the carrier, being it the element that has to fit with the docking system, a structure that in turn has to tackle problems not only in connection with a proper inclusion of the metallic elements, which should contribute to ensure an effective and reliable operation, improve safety by avoiding the release of metallic ions in the gastrointestinal environment, and be carried out with relatively simple capsule production steps. More generally speaking it is desirable that the capsule/carrier more successfully addresses fundamental issues such as the fabrication, assembly and filling in a reliable and certification-oriented way.

From a conceptual standpoint, the present invention then resides in a system that the docking group of which comprises two suitably distanced and independently controllable docking units for a capsule that correspondingly fits two ferromagnetic rings at positions displaced at respective axial ends of the capsule. From this, and from its constructional embodiments and implementations, a number of important advantages ensue, related not only with a remarkably safer, more stable and more reliable operation of the system, but also with the structure and production of the carrier that can be made safer, easier to be fabricated, assembled and filled.

### Brief description of the drawings

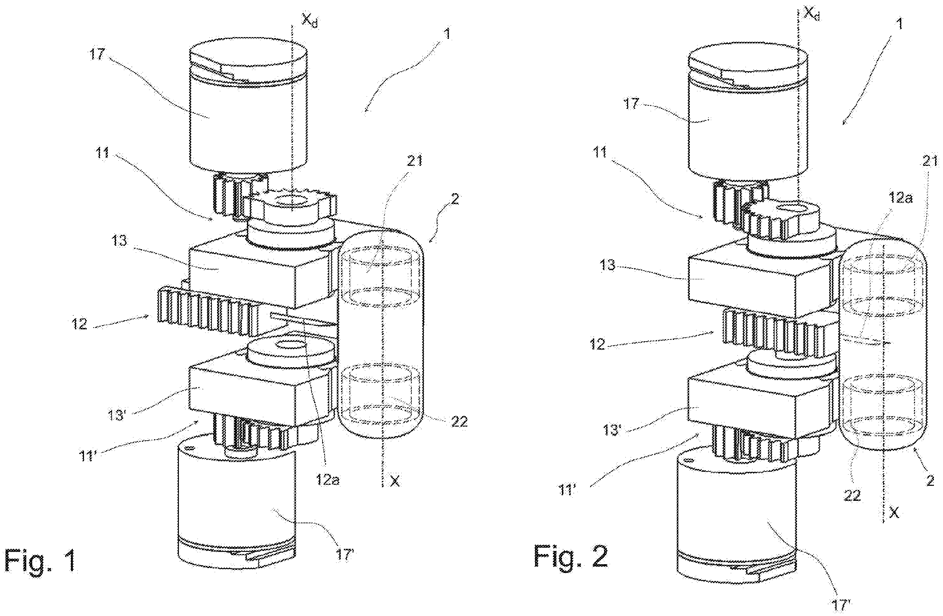

The characteristics and advantages of the device and system according to the present invention will be apparent from the following description of embodiments thereof, provided by way of non-limiting example with reference to the appended drawings wherein:
- figure 1 represents a schematic perspective view of a refilling station and substance carrier of the system, in a docking condition, with a punching needle in a retracted or rest position, parts being omitted for the sake of illustrative clarity;
- figure 2 shows in analogous manner the refilling station and the docked carrier with the punching needle in an advanced or punching position;
- figure 3 is a perspective view of a substance carrier capsule according to a first embodiment of the invention;
- figure 4 is a longitudinal axial section of the capsule of figure 3;
- figures 5a and 5b are exploded views of the capsule of figures 3 and 4, respectively seen in perspective and in a longitudinal axial section;
- figures from 6a to 6e show respective steps of an assembly procedure of the capsule in the first embodiment;
- figure 7 is a longitudinal axial section of a variant of the capsule of the first embodiment;
- figure 8 is an exploded view of the capsule of figure 7;
- figures from 9a to 9c show respective steps of an assembly procedure of the capsule in the variant of the preceding figures 7 and 8;

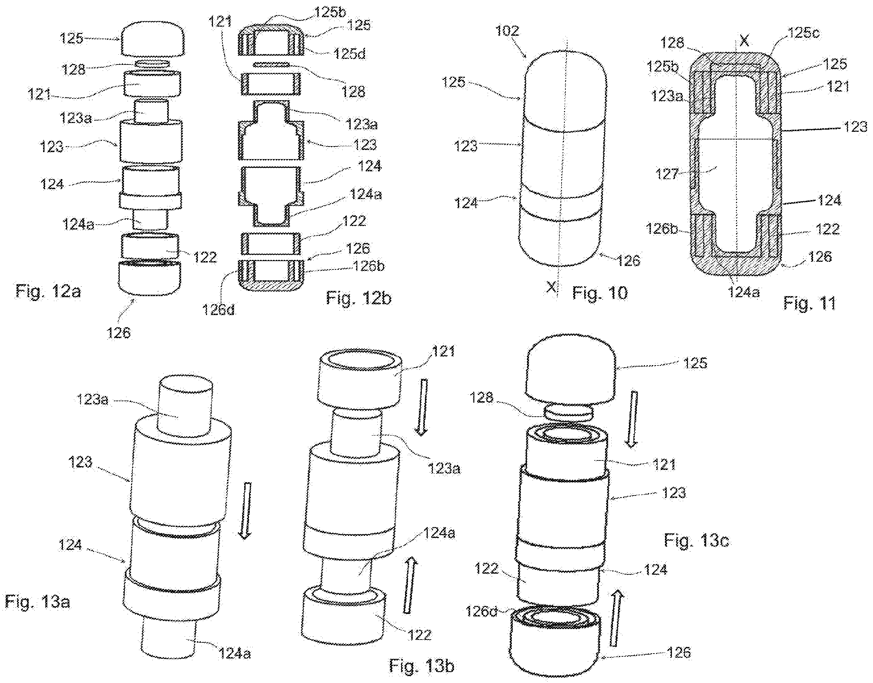

- figure 10 is a perspective view of a substance carrier capsule according to a second embodiment of the invention;
- figure 11 is a longitudinal axial section of the capsule of figure 10;
- figures 12a and 12b are exploded views of the capsule of figures 10 and 11, respectively seen in perspective and in a longitudinal axial section;
- figures from 13a to 13c show respective steps of an assembly procedure of the capsule in the second embodiment;
- figures 14a and 14b are perspective views elucidating possible filling methods of capsules according to the invention in case of an open capsule and in case of a closed capsule, respectively;
- figures 15a and 15b show schematically but in greater detail with respect to the depictions of figures 1 and 2 a docking unit and actuator of the docking group according to an embodiment of the invention, respectively in an inactive and in an active position;
- figures from 16a to 16c show in a top plan view respective possible variant arrangements of a switchable magnetic circuit of a docking unit, the arrangement in figure 16a corresponding to the embodiment in figures 15a and 15b;
- figures from 17a to 17h show via schematic perspective views, the unit actuators being omitted, the succession of steps of a capsule docking and release, including the refilling step through the punching needle, according to the invention and with a refilling station as in figures 1, 2, and 15a/15b;

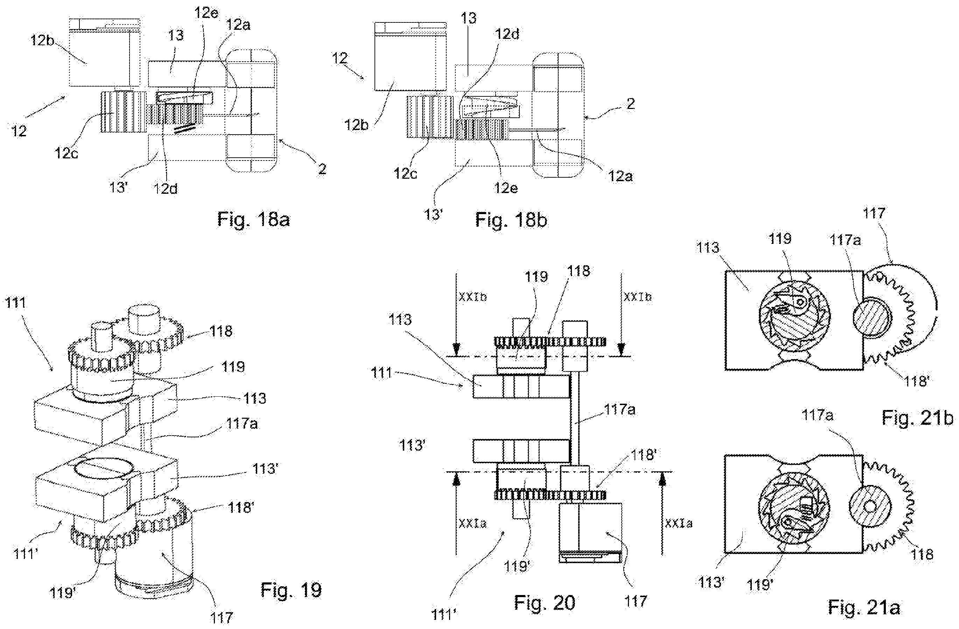

- figures 18a and 18b are side views of a refilling station (docking group and punching unit) as in the previous figures (the unit actuators being likewise omitted), with the punching needle in the advanced position at two respectively different punching points, the figures showing in fact a mechanism allowing a displacement of the needle crosswise with respect to the punching direction;
- figure 19 is a perspective view of a different embodiment of a docking group according to the invention, with the two units independently driven by a single actuator;
- figure 20 is a side view of the group of figure 19; and
- figure 21a and figures 21b are sectional views of the group taken respectively along the planes XXla and XXIB of figure 20.

### Detailed description of the Invention

With reference to the above figures, and in particular for the moment to figures 1 and 2, according to the invention, in an implanted infusion device of a system for the controlled administration of a substance, having the above mentioned characteristics as generally outlined in prior art document WO2012011132 and not shown in its entirety, a magnetic docking group of a refilling station 1 is provided, comprising two separate docking units 11, 11' placed at a proper distance, for docking with respective ferromagnetic rings 21, 22 arranged at the two axial ends of a substance carrier capsule 2. The refilling station 1 further comprises a punching unit 12, arranged between the two docking units 11, 11', and then adapted to punch with a punching needle 12a the capsule 2 at a central region between the capsule rings 21, 22.

Entering into further detail, starting from the capsule, and making reference to a first embodiment shown in figures from 3 to 9c, the capsule 2, elongated along an axis X, comprises two component shells 23, 24 designed to geometrically match each other by sliding telescopic engagement along the axis X, and form one cylindrical tubular body which creates a cavity 27 for the target substance (e.g., insulin). This internal cavity 27 of the carrier is in fact dedicated to host the hormone/drug or substance in general. It has a smooth internal surface, without sharp edges, to prevent hormone clotting (sharp surfaces act as triggers, e.g. for insulin aggregation, which is an undesired behavior).

Each shell comprises an axial end structure 23a, 24a at the end opposite to the mutual shell engagement end. In this embodiment the axial end structure 23a of one of the shells, here the shell 23 represented in top position, can have a central hole 23c to allow substance filling during the assembly, as in the variant of figures from 3 to 6e, or it can be closed as in the variant of figures from 8 to 9c where the slightly different end structure is indicated at the numeral 23a'. The mentioned end structures form respective annular slots 23b, 24b, around respective central and axially protruding pegs, for housing and integrating the two ferromagnetic rings 21, 22. The rings are then positioned and mutually distanced at respective axial ends of the capsule

Two caps 25, 26 geometrically match with respective end structures 23a, 24a to hermetically close the slots 23b, 24b, isolating the rings 21, 22 and shaping the capsule with rounded ends favoring ingestion and traveling along the gastrointestinal tract. To this purpose the cap has a mushroom shape with a dome surfaced head 25a, 26a that joins with the cylindrical outer surface of the body without forming sharp edges, and a tubular skirt portion 25b, 26b that fits within respective slots 23b, 24b at the inside of the rings 21, 22 (the bore of the skirt engaging with the central peg of the end structure), to block the rings in a snug-fit fashion.

The structure of the cap 25 closing the end structure 23a of the shell 23 that may be open or closed, depending on the variant, changes correspondingly. For the open-end structure of figures from 3 to 6e, the cap 25 integrally forms, opposite the outer dome surface of the head 25a, an axial protrusion 25c that closes hermetically the hole 23c. On the other hand, if the end structure 23a' is closed (figures from 8 to 9c), the inside of the cap is shaped with a recess 25d' so as to permit the insertion and housing of a septum disc 28 as clarified hereafter.

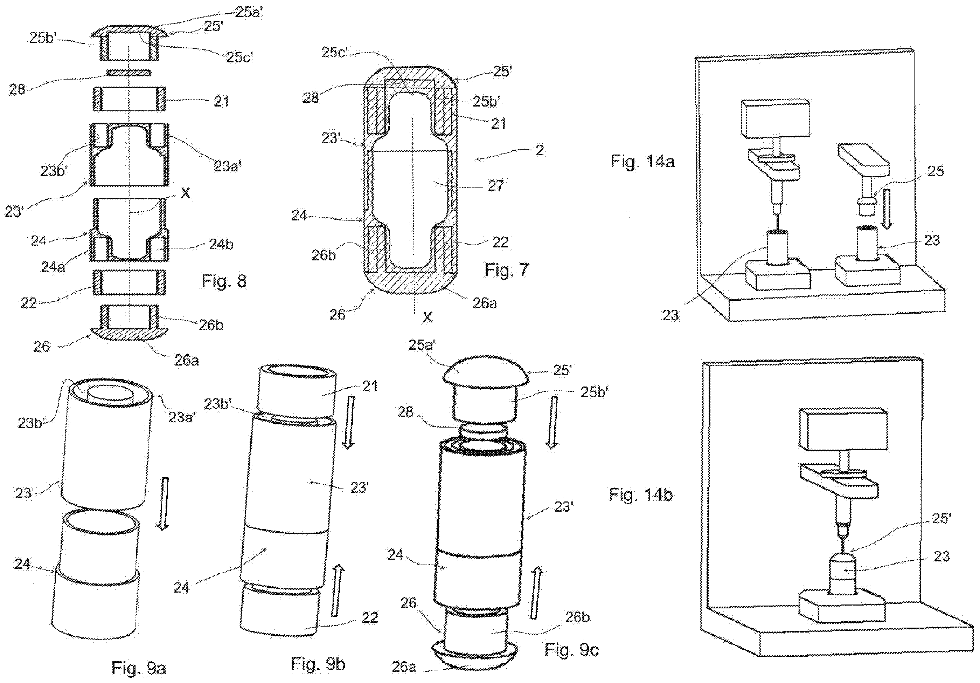

The assembly of the capsule body is a rather straightforward procedure that relies on the geometrical matching between the different components (self-explanatory figures from 6a to 6e and from 9a to 9c, and considering also figures 14a and 14b), coupled with subsequent sealing actions. In particular, the assembly steps can be summarized as follows.

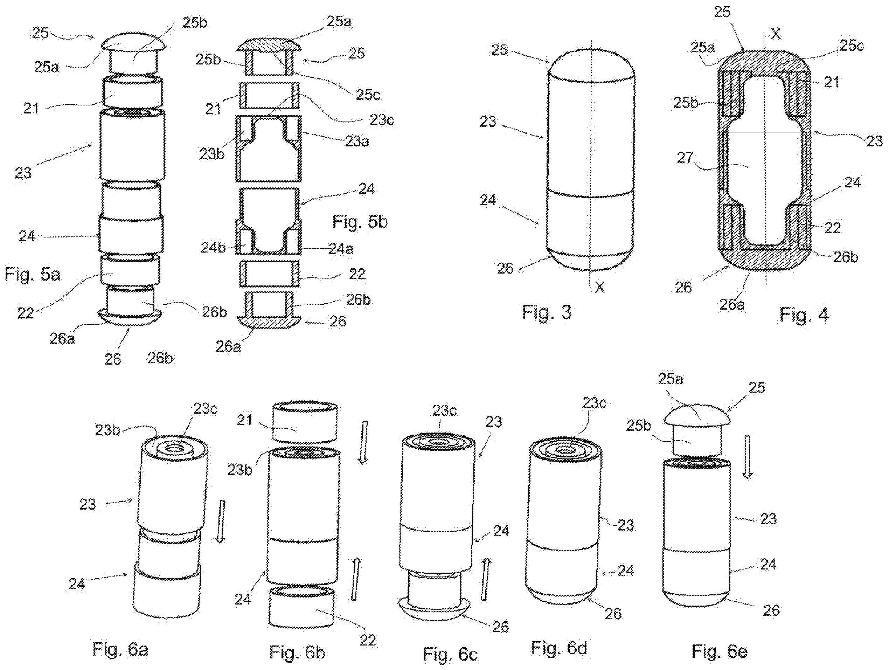

Firstly, the two carrier body components/shells 23, 24 are assembled together and sealed using a medical-grade adhesive (e.g. cyanoacrylates, cyclohexanone, etc.) or a direct bonding method (e.g. thermal welding with a laser), as in figures 6a and 9a.

The ferromagnetic rings 21, 22 are inserted into their dedicated slots 23b, 24b (figures 6b and 9b). First the lower cap 26 (i.e. in general the cap not involved with the substance filling procedure) is then made to match with the end structure 24a of the relative shell, entering the slot 24b with its skirt 26b, forming a curved external capsule shape and keeping the ring 22 within the body of the capsule, without leaving it exposed to the body fluids and tissues (figures 6c, 6d, 9c). Also this step can be carried out via sealing using a medical-grade adhesive or direct bonding process.

The other cap 25 is finally assembled analogously at the other end structure 23a, again to block ring 21 in the slot 23b, but with slightly different techniques dependent on the filling procedure. If the end structure 23a is open (figure 6d) the capsule can be filled with the substance at this stage, by holding the body of the capsule with known dedicated filling systems that allow open vial filling (figure 14a, left part of the image), then the cap 25 is inserted and assembled to form the final capsule shape structure (figure 6e, and image at the right of figure 14b). On the other hand, when the end structure 23a' of the shell 23' is closed (figure 9c), the cap 25' will be assembled without considering filling the capsule at this stage. The filling of the insulin (or substance in general) can be done later through a known standard filling system adapted to fill the closed capsule and eliminating the build-up pressure during substance dispensing (figure 14b). According to this, and to prevent any further leakage in and out of the capsule the self-healing material septum disc 28 is inserted and, as mentioned, the cap 25' is shaped to hold the septum disc 28 in the central recess 25c'. The puncture hole created by needle insertion may be closed using a biocompatible patch or using a direct bonding method to seal the hole without affecting the drug viability inside the capsule.

An alternative, second embodiment of a capsule 102 is shown in figures from 10 to 13c, where components corresponding to those of the first embodiment bear corresponding numerals in the hundreds and are not being described again in unnecessary detail. The two shells 123, 124 are here again hermetically closed thanks to the matching between the two parts, creating the cavity 127 that hosts the substance (hormone or drug in general). In this alternative design, the axial end structures 123a, 124a do not form the annular slots as in the previous case, but rather slots 125d, 126d housing the rings 121, 122 are formed in the respective caps 125, 126, and more precisely within the relative skirts 125b, 126b, duly thickened and wider (having the same outer diameter of the shells and developing in continuity with the same) with respect to the previous embodiment. The end structures 123a, 124a have accordingly a simplified shape, defining each a central peg that becomes engaged with the inner central bore of the cap skirt. As far as the filling end configuration is concerned, in this embodiment the preferred solution is the one with the septum disc 128 and relative recess 125c in the cap 125.

In both the proposed embodiments, the ideal capsule diameter and length may be around 12 mm and 26 mm, respectively, which are suitable for ingestion and traveling in the gastrointestinal tract. However, different dimensions can be devised, by properly scaling the single components. As already mentioned, it is worth noting that the final carrier configuration obtained from the assembly of the mentioned parts presents no protruding edges, thus favoring ingestion and travel and safe interaction with tissues.

The carrier body and caps are made of known materials featured by resistance to gastrointestinal fluids and suitable mechanical properties to withstand peristalsis. At the same time, they allow punching through the dedicated needle, to enable substance (e.g. insulin) transfer from the ingestible carrier to the implanted reservoir, possibly a collapse of the internal capsule volume during aspiration (that facilitates the procedure) and biocompatibility. Finally, they guarantee e.g. insulin stability for a reasonable amount of time (at least 12-24 h). Constitutive materials with these properties can be selected among (but they are not limited to) thermoplastic polyurethanes, thermoplastic elastomers, polyvinyl chloride, medical silicones, polydimethylsiloxane. The carrier body and caps can be fabricated with injection molding, 3D printing, or casting. All the mentioned techniques are compatible with the proposed materials and suitable for future mass production of certified products. The internal surface of the capsule may be coated with other materials/molecules, to enhance the stability of the hormone/drug contained in it.

The rings are made of materials reactive to the magnetic field which can be selected among ferromagnetic materials, ferromagnetic alloys, composite polymer with magnetic fillers or permanent magnets.

Overall, the proposed design prevents the hormone/drug to enter in touch with the magnetic rings, thus avoiding clotting or other adverse effects in terms of hormone/drug stability. Furthermore, the overall carrier structure is conceived to avoid substance contact with air and biological environment and to guarantee proper sealing without any leakage.

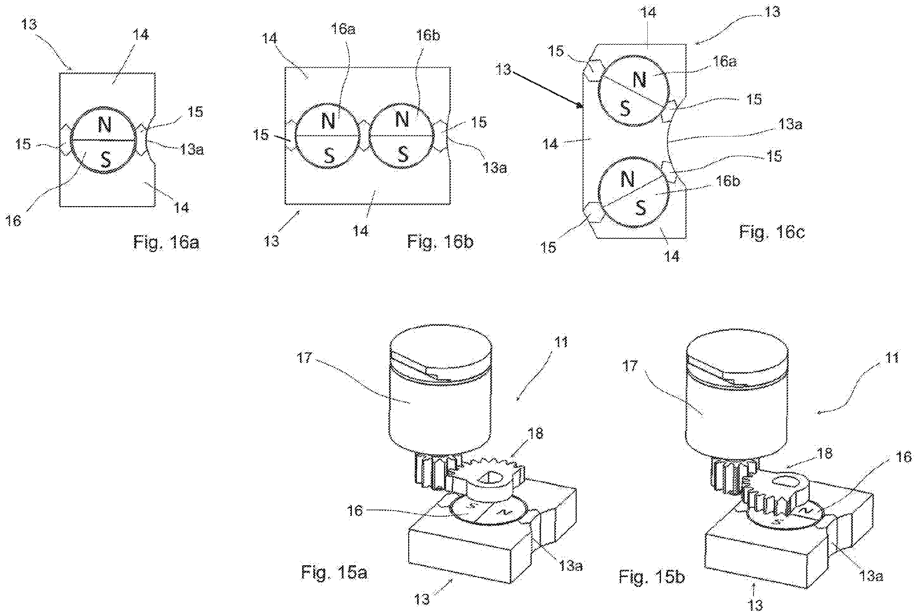

Considering now in greater detail the magnetic docking group 1, with specific reference also to the remaining figures starting from figure 15a onwards, each docking unit 11, 11' comprises a magnetic switchable circuit 13, 13' that can be independently activated, the two circuits providing respective docking points 13a, 13a' each adapted to dock with a respective ring and thus mutually spaced along a docking group axis X_{d} to the same extent as the two ferromagnetic rings of the capsule along the capsule axis X.

Each docking unit, with specific reference to figures 16a, 16b and 16c, can be based on switchable magnets or electromagnets, in a generally plate-shaped circuit that comprises ferromagnetic portions 14 and non-ferromagnetic portions 15 alternating along the periphery of the plate, at least one of the non-ferromagnetic portions 15 being outlined according to an arched cut-out edge defining said docking point 13a, geometrically matching with the periphery of the capsule at the height of the ring.

In this embodiment the unit further comprises one or more permanent magnets 16, with indicated N and S poles, possibly arranged in different configurations. While in figure 16a the arrangement provides a single permanent magnet 16, in figures 16b and 16c examples are shown in which two permanent magnets 16a, 16b are arranged so as to increase the attraction force.

The rotation of the magnet, for the sake of simplicity here reference is made to the single magnet arrangement as in figures 15a and 15b, is controlled by a rotational actuator 17 and relative transmission mechanism 18, for instance a geared transmission, to magnify the actuator output torque and to keep the circuits in the desired configuration without additional power consumption. In this way, the magnetic streamlines are readdressed in a way to stably dock the capsule ferromagnetic rings, in the switched-on configuration (figure 15b), and to turn the magnetic attraction force to almost zero in the switched-off configuration (figure 15a), thus achieving the undocking.

As mentioned, in an aspect of the invention two separate docking units are placed at a proper distance to enable docking of the two carrier rings. The activation of the two docking units can be controlled independently, e.g. via respective actuators 17, 17' and transmissions 18, 18' as proposed in the embodiment shown in figures 1 and 2, but other constructive solutions can be made use of, as clarified hereafter.

Figures from 17a to 17h elucidate the operation of the system, also showing the punching unit 12 with its own actuator 12b and pinon-rack gearing 12c, 12d for making the needle 12a move between a retracted/inactive position and an advanced/punching position. In this connection, it should be appreciated that the skilled person will obviously adapt the configuration of the communication and control unit of the infusion device, based on implementations such as the one in WO2012011132 and related technology that is known as such and need not be described in detail, to execute the operational step of the system (and any other functions provided by the system and outside the scope of the present invention).

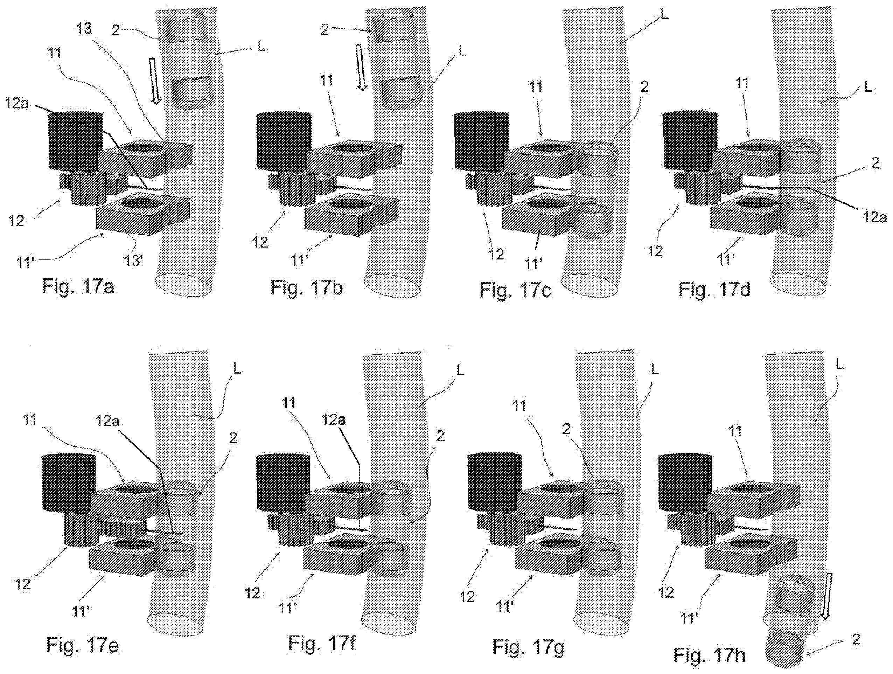

In figures 17a and 17b a capsule 2 approaches the refilling station 1 when traveling along a gastrointestinal tract lumen L, both the docking units being initially (figure 17a) in the switched-off position, and the punching unit 12 in the retracted position (inactive condition of the refilling station). According to an aspect of the invention, as the capsule gets closer, the bottom docking unit 11' is devised to be activated first (figure 17b) allowing the capture of the bottom capsule ring 22 (figure 17c). The success of this docking step can be monitored by the dedicated sensors included, as known or obviously adaptable, in the infusion device, being it clear that the bottom unit and the bottom ring are intended to be the downstream ones taking the travel path of the capsule as a reference.

As a subsequent step, the circuit of the upper unit 11 is activated to ensure stable docking of the capsule also on the upper/upstream ring 21 before punching (figure 17d). In the proposed configuration, the punching occurs at the center of the capsule, in between the two docking points thus preventing carrier tilting due to needle punching (figure 17e). The punching could also occur in slightly different points, but still included between the two ferromagnetic rings of the capsule, thus minimizing the momentum of the punching force. After completing the substance transfer/refilling step the needle retracts (figure 17f), and afterwards both the docking units are switched-off (figure 17g) to allow the release of the capsule (figure 17h).

Optionally, a mechanism allowing changing of the punching point can be implemented to minimize possible repeated damage to a specific point of the intestinal wall, which would thus have more time to recover/heal. A possible embodiment of this mechanism and relative operation is shown in figures 18a and 18b. The rotation of the magnet 16 (e.g. of the upper magnetic circuit 11) can be linked with a simple and elastically biased screw member 12e to move the transmission rack integral with the needle 12a in the height direction (an adjustment direction parallel to the docking group axis X_{d}). The screw member is configured with half pitch righthanded threads and half pitch lefthanded threads. So, by every 180° rotation of the magnet (equals to one activation and deactivation) the needle linear transmission rack 12d will move down e.g. of about 2 mm (figure 18a) and by 360° rotation the needle will come back up to the initial position (figure 18b). By this transition in height the needle can punch alternatively at an initial position and at a 2 mm lowered spot.

As mentioned, the independent/selective activation of the docking units can be driven also by alternative solutions to that of two actuators each driving its own docking unit. In fact, a single actuator, provided with an appropriate mechanism, may be used to drive both units, as per the embodiment of the docking group shown in figures from 19 to 21a This can allow saving space in the implanted system. This specific solution can be based on decoupling the clockwise and counter-clockwise rotation of a single actuator 117 to drive either magnetic circuits 111 and 111'. Accordingly, an actuator shaft 117a drives both transmission gearings 118, 118' of the magnetic circuits 111, 111' through respective ratchets 119, 119', each of them allowing transmissive engagement only in one direction of rotation, while remaining idle in the other direction. Clearly, the rachets are configured in a mutually opposed fashion, so that the engaging/active direction of rotation one of the two ratchets correspond to the idle direction of the other. However, other architectures (e.g., based on a higher number of independent actuators), but producing the same series of actions, can be equivalently devised by the skilled person.

It will be easily understood that according to the present invention the advantages of a reliable/stable docking of the carrier and of a carrier design that facilitates the fabrication, assembly, and filling procedures are attained concurrently. In fact, the combined new arrangement of the docking/punching system and of the carrier allows stable and reliable docking, capsule punching, and thus the reservoir refilling with the target substance.

As far as the docking procedures are concerned, the possibility of a sequential switching by two independently driven and properly spaced units permits to have the capsule reliably docked with a correct and precise positioning. The punching can then occur in turn with a reliable success and, being the punching unit arranged between the docking units, without the risk that an overturning momentum may cause the undocking of the carrier before the filling is completed (or even started). The rings at the axial ends of the capsule are advantageously embodied in a structure that makes them safely unexposed to the gastrointestinal fluids and in general to the tissue environment, and at the same time provides a multi-component solution that guarantees a reliable fabrication and assembly of the carrier thus compatible with industrial production and suitable for future certification stages.

The present invention has been described with reference to preferred embodiments thereof. Variations and/or modifications can be brought to the invention without thereby departing from the scope of the invention itself as defined by the attached claims.

## Claims

1. An infusion device for the administration of a substance, implantable in the peritoneal cavity of a patient, comprising: - a communication and control unit to manage data arriving from a monitoring unit for the substance release; - energy storage means; - a refilling station (1) controlled by said control unit to refill said infusion device with said substance and comprising a docking group for the magnetic docking of a substance carrier of the administered substance, in the form of a capsule (2) elongated along a capsule elongation axis (X), to be ingested by the patient to reach passively an intestinal lumen, made of perforable material, resistant to the gastro-intestinal fluids and with two ferromagnetic ring inserts (21, 22), and a punching unit (12) for drawing the substance from the capsule; the device being **characterized in that** said docking group comprises two independently controllable docking units (11, 11'), mutually distanced along a docking group axis (X_{d}), said punching unit (12) being arranged between the docking units (11, 11').

2. The infusion device according to claim 1, wherein each docking unit (11, 11') comprises a magnetic switchable circuit (13, 13'), adapted to be switched on and off to respectively dock and release said capsule upon rotation of at least one permanent magnet, and an actuator (17, 17') to independently drive said circuit.

3. The infusion device according to claim 1, wherein each docking unit (111, 111') comprises a magnetic switchable circuit (113, 113'), adapted to be switched on and off to respectively dock and release said capsule upon rotation of at least one permanent magnet (16), said docking group comprising a single actuator (117) to independently and selectively drive either circuit of the units.

4. The infusion device according to claim 3, wherein said single actuator (117) drives said circuits (111, 111') through rotation of a single shaft (117a) and respective ratchet means (119, 119'), each allowing transmissive engagement only in one direction of rotation, while remaining idle in the other direction, the rachet means (119, 119') being configured in a mutually opposed fashion, so that the engaging/active direction of rotation one of the two ratchets correspond to the idle direction of the other.

5. The infusion device according to any of claims 2 to 4, wherein said punching unit comprises a needle (12a) adapted to punch said capsule at a punching point, and a mechanism configured to change the position of the punching point in an adjustment direction parallel to said docking group axis (X_{d}).

6. The infusion device according to claim 5, wherein said punching unit comprises a needle actuator (12b) and a pinon and rack gearing (12c, 12d) between the needle actuator (12b) and said needle (12a) for making the latter move between a retracted or inactive position and an advanced or punching position, said mechanism comprising a screw member (12e) linking the position of said rack (12d) in said adjustment direction with the rotation of said at least one magnet (16), said screw member being configured with half pitch righthanded threads and half pitch lefthanded threads.

7. The infusion device according to any of the previous claims, wherein said communication and control unit is configured to: in an inactive condition of the refilling station (1), keeping both said docking units (11, 11') in a switched-off position, and said punching unit (12) in a retracted position; as a capsule approach is detected, switching on a docking unit (11') located downstream considering the travel path of the capsule (2) to cause it to dock with a downstream end ring (22) of the capsule; after docking the downstream ring (22) of the capsule, switching on the other docking unit (11) and cause it to dock with the other capsule ring (21); activating said punching unit (12) to punch the capsule (2); after completing the substance transfer/refilling, deactivating the punching unit (12); switching off both the docking units (11, 11') to release the capsule (2).

8. A system for the controlled administration of a substance from a human-body-implanted infusion device, the system comprising:
• an implantable monitoring unit for the monitoring of the administered substance;
• an infusion device according to any of the claims from 1 to 7;
• a substance carrier capsule (2) adapted to dock with said magnetic docking group of the infusion device, the capsule being elongated along a capsule elongation axis (X), made of perforable material, resistant to the gastro-intestinal fluids, and fitting two ferromagnetic ring inserts (21, 22) at positions displaced towards respective axial ends of the capsule (2), the capsule further comprising: two component shells (23, 24) geometrically matching each other by mutual sliding engagement along said capsule elongation axis (X) to form a cylindrical tubular body which creates a cavity (27) for said substance, each shell (23, 24) comprising an axial end structure (23a, 24a) at an end opposite to a mutual shell engagement end; two caps (25, 26) configured for geometrically matching arrangement with respective end structures; wherein said matching arrangement between the caps (25, 26) and the end structures (23, 24) forms respective annular closed slots (23b, 24b) for hermetically housing and isolating said two ferromagnetic ring inserts (21, 22) positioned and mutually distanced at respective axial ends of the capsule (2).

9. The system according to claim 8, wherein said end structures form said respective annular slots (23b, 24b), around respective central and axially protruding pegs, said two caps (25, 26) having each a mushroom shape with a dome surfaced head (25a, 26a) that joins with an outer surface of said tubular body without forming sharp edges, and a tubular skirt portion (25b, 26b) that fits within respective slots (23b, 24b) at the inside of the rings (21, 22) to block the rings in a snug-fit fashion.

10. The system according to claim 8, wherein said caps (125, 126) form said respective annular slots (125d, 126d) within respective tubular skirt portions (125b, 126b) having the same outer diameter of said shells (123, 124) and developing in continuity with the same, the end structures (123a, 124a) defining each a central peg that becomes engaged with an inner central bore of the cap skirt portion.

11. The system according to claim 9 or 10, wherein at least one of said end structures has a central hole (23c), the corresponding cap (25) integrally forming an axial protrusion (25c).

12. The system according to claim 9 or 10, wherein one of the caps is shaped with a recess (25d') for the insertion and housing of a self-healing material septum disc (28).

## Patentansprüche

1. Infusionsvorrichtung für die Verabreichung einer Substanz, die in die Bauchhöhle eines Patienten implantiert werden kann, umfassend: - eine Kommunikations- und Steuereinheit zur Verwaltung der von einer Überwachungseinheit für die Stofffreisetzung eingehenden Daten; - Energiespeichermittel; - eine Nachfüllstation (1), die von der Steuereinheit gesteuert wird, um die Infusionsvorrichtung mit dem Stoff nachzufüllen, und die eine Andockgruppe für das magnetische Andocken eines Stoffträgers des verabreichten Stoffes in Form einer Kapsel (2) umfasst, die sich entlang einer Kapseldehnungsachse (X) erstreckt, die vom Patienten eingenommen werden soll, um passiv in ein Darmlumen zu gelangen, und die aus einem perforierbaren Material besteht, das gegenüber den gastrointestinalen Fluiden beständig ist und zwei ferromagnetische Ringeinsätze (21, 22) aufweist, sowie eine Stanzeinheit (12) zum Herausziehen des Stoffes aus der Kapsel; die Vorrichtung **dadurch gekennzeichnet ist, dass** die Andockgruppe zwei unabhängig steuerbare Andockeinheiten (11, 11') umfasst, die entlang einer Andockgruppenachse (X_{d}) voneinander beabstandet sind, wobei die Stanzeinheit (12) zwischen den Andockeinheiten (11, 11') angeordnet ist.

2. Infusionsvorrichtung nach Anspruch 1, wobei jede Andockeinheit (11, 11') eine magnetische schaltbare Schaltung (13, 13') umfasst, die geeignet ist, ein- und ausgeschaltet zu werden, um die Kapsel bei Drehung mindestens eines Dauermagneten anzudocken bzw. freizugeben, und einen Aktuator (17, 17'), um die Schaltung unabhängig zu betreiben.

3. Infusionsvorrichtung nach Anspruch 1, wobei jede Andockeinheit (111, 111') eine magnetische schaltbare Schaltung (113, 113') umfasst, die geeignet ist, um ein- und ausgeschaltet zu werden, um die Kapsel bei Drehung mindestens eines Permanentmagneten (16) anzudocken bzw. freizugeben, wobei die Andockgruppe einen einzelnen Aktuator (117) umfasst, um unabhängig und selektiv jede Schaltung der Einheiten anzutreiben.

4. Infusionsvorrichtung nach Anspruch 3, wobei der einzelne Aktuator (117) die Schaltungen (111, 111') durch Drehung einer einzelnen Welle (117a) und entsprechender Ratschenmittel (119, 119') antreibt, die jeweils einen übertragenden Eingriff nur in einer Drehrichtung ermöglichen, während sie in der anderen Richtung inaktiv bleiben, wobei die Ratschenmittel (119, 119') in einer einander gegenüberliegenden Weise konfiguriert sind, sodass die eingreifende/aktive Drehrichtung einer der beiden Ratschen der Leerlaufrichtung der anderen entspricht.

5. Infusionsvorrichtung nach einem der Ansprüche 2 bis 4, wobei die Stanzeinheit eine Nadel (12a) umfasst, die geeignet ist, um die Kapsel an einem Stanzpunkt zu stanzen, und einen Mechanismus, der konfiguriert ist, um die Position des Stanzpunktes in einer Einstellrichtung parallel zur Andockgruppenachse (X_{d}) zu verändern.

6. Infusionsvorrichtung nach Anspruch 5, wobei die Stanzeinheit einen Nadelaktuator (12b) und ein Zahnstangengetriebe (12c, 12d) zwischen dem Nadelaktuator (12b) und der Nadel (12a) umfasst, um letztere zwischen einer zurückgezogenen oder inaktiven Position und einer vorgeschobenen oder stanzenden Position zu bewegen, wobei der Mechanismus ein Schraubenelement (12e) umfasst, das die Position der Zahnstange (12d) in der Einstellrichtung mit der Drehung des mindestens einen Magneten (16) verbindet, wobei das Schraubenelement mit einem Rechtsgewinde mit halber Steigung und einem Linksgewinde mit halber Steigung ausgeführt ist.

7. Infusionsvorrichtung nach einem der vorstehenden Ansprüche, wobei die Kommunikations- und Steuereinheit konfiguriert ist zum: Halten der beiden Andockstationen (11, 11') in einem inaktiven Zustand der Nachfüllstation (1) in einer ausgeschalteten Position und der Stanzeinheit (12) in einer zurückgezogenen Position; wenn eine Annäherung der Kapsel erkannt wird, Einschalten einer Andockeinheit (11'), die unter Berücksichtigung des Bewegungspfads der Kapsel (2) stromabwärts angeordnet ist, um sie zum Andocken an einen stromabwärts gelegenen Endring (22) der Kapsel zu veranlassen; nach dem Andocken des stromabwärts gelegenen Rings (22) der Kapsel, Einschalten der anderen Andockeinheit (11) und deren Andocken an den anderen Kapselring (21); Aktivieren der Stanzeinheit (12) zum Stanzen der Kapsel (2); nach dem Beenden der Stoffübertragung/Nachfüllung Deaktivieren der Stanzeinheit (12); Ausschalten der beiden Andockeinheiten (11, 11') zur Freigabe der Kapsel (2).

8. System zur gesteuerten Verabreichung eines Stoffes aus einer in den menschlichen Körper implantierten Infusionsvorrichtung, das System umfassend:
• eine implantierbare Überwachungseinheit zum Überwachen des verabreichten Stoffes;
• eine Infusionsvorrichtung nach einem der Ansprüche 1 bis 7;
• eine Stoffträgerkapsel (2), die zum Andocken an die magnetische Andockgruppe der Infusionsvorrichtung geeignet ist, wobei die Kapsel entlang einer Kapseldehnungsachse (X) langgestreckt ist, aus perforierbarem Material besteht, gegenüber den gastrointestinalen Fluiden beständig ist und zwei ferromagnetische Ringeinsätze (21, 22) an Positionen aufweist, die zu den jeweiligen axialen Enden der Kapsel (2) hin versetzt sind, die Kapsel ferner umfassend: zwei Komponentenschalen (23, 24), die durch gegenseitigen Gleiteingriff entlang der Kapseldehnungsachse (X) geometrisch zueinander passen, um einen zylindrischen röhrenförmigen Körper zu bilden, der einen Hohlraum (27) für den Stoff bildet, wobei jede Schale (23, 24) eine axiale Endstruktur (23a, 24a) an einem Ende umfasst, das einem Ende des gegenseitigen Schaleneingriffs gegenüberliegt; zwei Kappen (25, 26), die für eine geometrisch passende Anordnung mit den jeweiligen Endstrukturen konfiguriert sind; wobei die übereinstimmende Anordnung zwischen den Kappen (25, 26) und den Endstrukturen (23, 24) jeweils ringförmige geschlossene Schlitze (23b, 24b) zur hermetischen Aufnahme und Isolierung der beiden ferromagnetischen Ringeinsätze (21, 22) bildet, die an den jeweiligen axialen Enden der Kapsel (2) positioniert und voneinander beabstandet sind.

9. System nach Anspruch 8, wobei die Endstrukturen die jeweiligen ringförmigen Schlitze (23b, 24b) um die jeweiligen zentralen und axial vorstehenden Zapfen bilden, wobei die beiden Kappen (25, 26) jeweils eine Pilzform mit einem Kopf (25a, 26a) mit gewölbter Oberfläche, der mit einer äußeren Oberfläche des rohrförmigen Körpers ohne Bildung scharfer Kanten verbunden ist, und einen rohrförmigen Schürzenabschnitt (25b, 26b) aufweisen, der in entsprechende Schlitze (23b, 24b) an der Innenseite der Ringe (21, 22) passt, um die Ringe in einer passgenauen Weise zu blockieren.

10. System nach Anspruch 8, wobei die Kappen (125, 126) die jeweiligen ringförmigen Schlitze (125d, 126d) innerhalb jeweiliger rohrförmiger Schürzenabschnitte (125b, 126b) bilden, die den gleichen Außendurchmesser der Schalen (123, 124) aufweisen und sich in Kontinuität mit diesen entwickeln, wobei die Endstrukturen (123a, 124a) jeweils einen zentralen Zapfen definieren, der in eine innere zentrale Bohrung des Kappenschürzenabschnitts eingreift.

11. System nach Anspruch 9 oder 10, wobei mindestens eine der Endstrukturen ein zentrales Loch (23c) aufweist, wobei die entsprechende Kappe (25) einstückig einen axialen Vorsprung (25c) bildet.

12. System nach Anspruch 9 oder 10, wobei eine der Kappen mit einer Vertiefung (25d') zum Einsetzen und Aufnehmen einer Septumscheibe (28) aus selbstheilendem Material geformt ist.

## Revendications

1. Dispositif de perfusion pour l'administration d'une substance, implantable dans la cavité péritonéale d'un patient, comprenant : - une unité de communication et de commande pour gérer des données provenant d'une unité de surveillance de la libération de substance ;
- un moyen de stockage d'énergie ; - une station de remplissage (1) commandée par ladite unité de commande pour recharger ledit dispositif de perfusion avec ladite substance et comprenant un groupe d'amarrage pour l'amarrage magnétique d'un support de substance de la substance administrée, sous la forme d'une capsule (2) allongée le long d'un axe d'allongement de capsule (X), à ingérer par le patient pour atteindre passivement une lumière intestinale, faite de matériau perforé, résistant aux fluides gastro-intestinaux et ayant deux inserts annulaires ferromagnétiques (21, 22), et une unité de perforation (12) permettant d'aspirer la substance de la capsule ; le dispositif étant **caractérisé en ce que** ledit groupe d'amarrage comprend deux unités d'amarrage (11, 11') commandables indépendamment, mutuellement à distance le long d'un axe de groupe d'amarrage (X_{d}), ladite unité de perforation (12) étant agencée entre les unités d'amarrage (11, 11').

2. Dispositif de perfusion selon la revendication 1, dans lequel chaque unité d'amarrage (11, 11') comprend un circuit magnétique commutable (13, 13'), adapté pour être allumé et éteint pour respectivement amarrer et libérer ladite capsule lors de la rotation d'au moins un aimant permanent, et un actionneur (17, 17') pour entraîner indépendamment ledit circuit.

3. Dispositif de perfusion selon la revendication 1, dans lequel chaque unité d'amarrage (111, 111') comprend un circuit magnétique commutable (113, 113'), adapté pour être allumé et éteint pour respectivement amarrer et libérer ladite capsule lors d'une rotation d'au moins un aimant permanent (16), ledit groupe d'amarrage comprenant un actionneur unique (117) pour entraîner indépendamment et sélectivement l'un ou l'autre circuit des unités.

4. Dispositif de perfusion selon la revendication 3, dans lequel ledit actionneur unique (117) entraîne lesdits circuits (111, 111') par rotation d'un arbre unique (117a) et de moyens à cliquet (119, 119') respectifs, chacun permettant une mise en prise transmissive uniquement dans un sens de rotation, tout en restant au repos dans l'autre sens, les moyens à cliquet (119, 119') étant conçus d'une manière mutuellement opposée, de sorte que le sens de rotation/actif de l'un des deux cliquets correspond au sens de repos de l'autre.

5. Dispositif de perfusion selon l'une quelconque des revendications 2 à 4, dans lequel ladite unité de perforation comprend une aiguille (12a) adaptée pour perforer ladite capsule au niveau d'un point de perforation, et un mécanisme conçu pour modifier la position du point de perforation dans une direction d'ajustement parallèle audit axe de groupe d'amarrage (X_{d}).

6. Dispositif de perfusion selon la revendication 5, dans lequel ladite unité de perforation comprend un actionneur d'aiguille (12b) et un engrenage à pignon et crémaillère (12c, 12d) entre l'actionneur d'aiguille (12b) et ladite aiguille (12a) permettant de faire se déplacer cette dernière entre une position rétractée ou inactive et une position avancée ou de perforation, ledit mécanisme comprenant un élément vis (12e) reliant la position de ladite crémaillère (12d) dans ladite direction d'ajustement à la rotation dudit au moins un aimant (16), ledit élément vis étant conçu avec des filetages à droite à demi-pas et des filetages à gauche à demi-pas.

7. Dispositif de perfusion selon l'une quelconque des revendications précédentes, dans lequel ladite unité de communication et de commande est configurée pour : dans un état inactif de la station de remplissage (1), maintenir lesdites deux unités d'amarrage (11, 11') dans une position allumée, et ladite unité de perforation (12) dans une position rétractée ; lorsqu'une approche de capsule est détectée, mettre en marche une unité d'amarrage (11') située en aval compte tenu de la trajectoire de déplacement de la capsule (2) pour l'amarrer à un anneau d'extrémité aval (22) de la capsule ; après l'amarrage de l'anneau aval (22) de la capsule, mettre en marche l'autre unité d'amarrage (11) et l'amarrer à l'autre anneau de capsule (21) ; activer ladite unité de perforation (12) pour perforer la capsule (2) ; après achèvement du transfert/remplissage de substance, désactiver l'unité de perforation (12) ; éteindre les deux unités d'amarrage (11, 11') pour libérer la capsule (2).

8. Système pour l'administration commandée d'une substance à partir d'un dispositif de perfusion implanté dans le corps humain, le système comprenant :
• une unité de surveillance implantable pour la surveillance de la substance administrée ;
• un dispositif de perfusion selon l'une quelconque des revendications 1 à 7 ;
• une capsule de support de substance (2) adaptée pour s'amarrer audit groupe d'amarrage magnétique du dispositif de perfusion, la capsule étant allongée le long d'un axe d'allongement de capsule (X), faite de matériau perforé, résistant aux fluides gastro-intestinaux, et ajustant deux inserts annulaires ferromagnétiques (21, 22) au niveau de positions déplacées vers des extrémités axiales respectives de la capsule (2), la capsule comprenant en outre : deux enveloppes constitutives (23, 24) s'adaptant géométriquement l'une à l'autre par mise en prise coulissante mutuelle le long dudit axe d'allongement de capsule (X) pour former un corps tubulaire cylindrique qui crée une cavité (27) pour ladite substance, chaque enveloppe (23, 24) comprenant une structure d'extrémité axiale (23a, 24a) au niveau d'une extrémité opposée à une extrémité de mise en prise mutuelle d'enveloppe ; deux capuchons (25, 26) conçus pour s'adapter géométriquement à des structures d'extrémité respectives ; dans lequel ledit agencement d'adaptation entre les capuchons (25, 26) et les structures d'extrémité (23, 24) forme des fentes annulaires fermées respectives (23b, 24b) permettant de recevoir et isoler hermétiquement lesdits deux inserts annulaires ferromagnétiques (21, 22) positionnés et mutuellement à distance au niveau d'extrémités axiales respectives de la capsule (2).

9. Système selon la revendication 8, dans lequel lesdites structures d'extrémité forment lesdites fentes annulaires respectives (23b, 24b), autour de chevilles respectives centrales et axialement en saillie, lesdits deux capuchons (25, 26) ayant chacun une forme de champignon avec une tête à surface de dôme (25a, 26a) qui se joint à une surface externe dudit corps tubulaire sans former de bords tranchants, et une partie de jupe tubulaire (25b, 26b) qui s'insère au sein de fentes respectives (23b, 24b) à l'intérieur des anneaux (21, 22) pour bloquer les anneaux de manière ajustée.

10. Système selon la revendication 8, dans lequel lesdits capuchons (125, 126) forment lesdites fentes annulaires respectives (125d, 126d) au sein de parties de jupe tubulaire respectives (125b, 126b) ayant le même diamètre externe que lesdites enveloppes (123, 124) et se développant en continuité avec celles-ci, les structures d'extrémité (123a, 124a) définissant chacune une cheville centrale qui vient en prise avec un alésage central interne de la partie de jupe de capuchon.

11. Système selon la revendication 9 ou 10, dans lequel au moins l'une desdites structures d'extrémité a un trou central (23c), le capuchon (25) correspondant formant d'un seul tenant une saillie axiale (25c).

12. Système selon la revendication 9 ou 10, dans lequel l'un des capuchons est formé d'un évidement (25d') pour l'insertion et la réception d'un disque de septum en matériau auto-régénérant (28).
